(19) Europäisches Patentamt
European Patent Office
Office européen des brevets

(11) Publication number: **0 095 206**
**B2**

(12) **NEW EUROPEAN PATENT SPECIFICATION**

(45) Date of publication of the new patent specification: **12.10.88**

(51) Int. Cl.⁴: **C 07 C 49/713,** C 25 B 3/02, C 07 C 45/28

(21) Application number: **83200689.4**

(22) Date of filing: **16.05.83**

(54) **Process for the synthesis of 2,4,6-trimethyl-4-hydroxycyclohexa-2,5-dien-1-one.**

(30) Priority: **20.05.82 IT 2138582**

(43) Date of publication of application:
**30.11.83 Bulletin 83/48**

(45) Publication of the grant of the patent:
**11.09.85 Bulletin 85/37**

(45) Mention of the opposition decision:
**12.10.88 Bulletin 88/41**

(84) Designated Contracting States:
**BE DE FR GB NL**

(56) References cited:
**FR-A-2 368 458**

**CHEMICAL ABSTRACTS, vol. 93, no. 2, July 1980, page 484, no. 33968v, Columbus, Ohio, USA**

**F.Fichter in "Organische Elektrochemie", Verlag Theodor Steinkopff, 1942, p. 107**
**J.Chem.Soc., Perkin Trans 1 (1973), pp. 2337-45**

(73) Proprietor: **ANIC S.p.A.**
**Via Ruggero Settimo, 55**
**I-90139 Palermo (IT)**

(72) Inventor: **Oberrauch, Ermanno**
**Via S. Marcellina 4**
**I-20125 Milan (IT)**
Inventor: **Eberson, Lennart**
**Dragonwägen 4**
**S-222 39 Lund (SE)**

(74) Representative: **Roggero, Sergio et al**
**Ing. Barzanò & Zanardo Milano S.p.A.**
**Via Borgonuovo 10**
**I-20121 Milano (IT)**

**EP 0 095 206 B2**

Courier Press, Leamington Spa, England.

**Description**

This invention relates to a process for preparing 2,4,6-trimethyl-4-hydroxycyclohexa-2,5-dien-1-one (indicated hereinafter by the symbol CHD) by electrochemically oxidising 1,3,5-trimethylbenzene (mesitylene) under suitable experimental conditions.

The electrochemical preparation of CHD is described in the scientific literature (see Chemical Communications 1971 p. 2337) and the patent literature (see Japanese patent application 80/20745). However, these methods use 2,4,6-trimethylphenol (mesitol) as their starting material, this being a high cost compound which is not easily obtainable commercially in industrial quantities.

We have now discovered that CHD can be synthesised electrochemically from mesitylene, which is an economical aromatic hydrocarbon which is widely available.

This represents a result which could not be foreseen because the side chains of alkylbenzenes are generally more easily oxidised than the aromatic nucleus. The pecularity of this reaction comprising the oxidation of mesitylene to CHD therefore represents an essential characteristic of the invention.

The method to be followed in effecting this process represents a further essential characteristic of the invention, because by means of its application it is possible by oxidising mesitylene to obtain CHD as the main product instead of other compounds.

From the aforegoing, it is apparent that the present invention relates to a reaction which has never before been described, and to the most suitable means for effecting it. This new reaction can be represented schematically as follows:

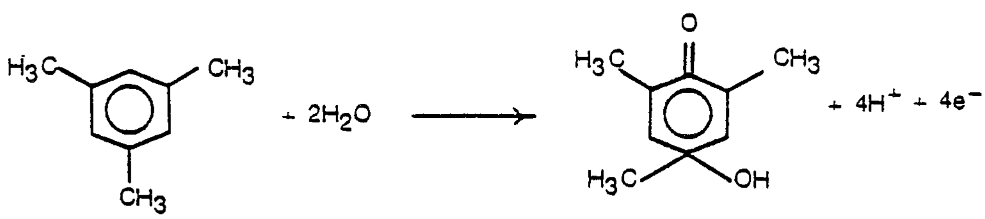

(mesitylene) (CHD)

This scheme is valid for the electrochemical attainment of the present invention. The removal of four electrons from the mesitylene molecule takes place in the first case by the action of the electric current on the anode surface of an electrolytic cell, and in the second case by the action of a suitable oxidising agent in a common chemical reactor.

Both the electrochemical and chemical reactions have been studied in order to determine the nature of the by-products which accompany the formation of the CHD.

The following by-products have been identified, generally in small quantities: 2,6-dimethylbenzoquinone; 3,5-dimethylbenzaldehyde; mesitol; 3,5-dimethylbenzylalcohol; 2,4,6-trimethylbenzaldehyde. The reaction mixtures also contain polymer compounds (which are however easily separable as a distillation residue) deriving from oxidation coupling reactions.

The CHD and the various aforesaid by-products have been identified by the normal analytical methods ($^1H$ NMR, mass spectrometry) and by using commercial or purposely prepared comparison compounds.

The details of the method according to the present invention, namely the electrochemical method, are described hereinafter.

The synthesis of CHD according to the present invention is effected by the electrochemical method.

In this case, CHD is obtained as the main reaction product by the particular choice of the solvent, the support electrolyte, the anode material and the operating conditions.

In this respect, the electrochemical oxidation of mesitylene, as described in literature (see for example Chemical Abstracts Vol. 87 No. 13442) is usually carried out in the absence of organic solvents, and leads to the formation of 3,5-dimethylbenzaldehyde as the main product.

According to the invention, a process is disclosed for preparing 2,4,6-trimethyl-4-hydroxycyclohexa-2,5-diene-1-one, which is characterized in that mesitylene is oxidized electrolytically, the reaction being carried out within a cell equipped with a diaphragm separating the anode compartment from the cathode compartment, wherein the anolite consists of acetonitrile, water and a mineral acid or a mixture of alkali metal salts and tetraalkyl ammonium.

The anodes used are constituted by lead or its alloys (particularly with Ag, Sb, Sn or Tl) or by lead dioxide deposited on various materials such as lead, lead alloys (particularly with Ag, Sb, Sn or Tl), titanium or graphite, or by graphite, carbon or materials deriving therefrom.

Lead dioxide or graphite anodes are preferably used.

Lead, steel or other metals or alloys which are inert under the operating conditions can be used as the cathodes.

The anodic electrolyte solution generally comprises several compounds. A first compound is an organic solvent, the purpose of which is to increase the mesitylene solubility in the reaction medium. It is usually aprotic and miscible with water. Solvent suitable for this purpose is acetonitrile.

A second component of the anodic electrolyte solution is water, present to the extent of 1 part by volume for every 0.5—100 parts by volume of solvent, and more particularly for every 2—10 parts by volume of solvent.

A third constituent of the anodic electrolyte solution is a mineral acid, or a suitable mixture of alkaline salts and tetraalkylammonium. Suitable acids for this purpose are of the non-oxidising type, and having a poorly nucleophilic anion, such as $H_2SO_4$, $HClO_4$, $HBF_4$, $H_3PO_4$. The most suitable salts are those derived from the same acids, ie sulphates, perchlorates, fluoroborates, alkaline phosphates and tetraalkylammonium, including their mixtures. Of the said electrolytes, $H_2SO_4$ and phosphate-fluoroborate mixtures are preferably used. A fourth constituent of the anodic electrolyte mixture can in certain cases be added in order to control the acidity of the anodic solution during the electrolysis. Effective substances for this purpose are alkaline and alkaline-earth bicarbonates and carbonates, and particularly $NaHCO_3$ and $BaCO_3$.

The mesitylene concentration in the anode compartment can vary within wide limits. The present invention in fact comprises processes in which the mesitylene is completely dissolved, and those in which the electrolysis is carried out in a two-phase hydro-organic system.

The mesitylene is used in a volumetric ratio relative to the anodic electrolyte solution which varies between 1:200 and 1:1, and more commonly between 1:100 and 1:10.

The current density used is suitably chosen on the basis of the electrode-electrolyte system, and the working conditions. Generally, current densities of between 5 mA/cm$^2$ and 150 mA/cm$^2$, and more commonly between 7 and 100 mA/cm$^2$, have proved suitable for the purpose.

The cathodic electrolyte solution comprises an aqueous or hydroorganic solution of a strong electrolyte. As the cathodic reaction consists simply in the evolving of hydrogen and does not interfere with the process which characterises the present invention, there is a wide choice. Thé use of the same acid (or of the acid corresponding to one of the salts) as is present in the anode compartment is particularly convenient.

The operating temperature is chosen between 0°C and 50°C. In most cases, it is preferable to maintain a temperature of between 5°C and 25°C.

The current quantity used for the electrolysis (with respect to the theoretical quantity of 4 Faradays per mole of mesitylene necessary for producing one mole of CHD) can be chosen between 1 and 8 Faradays per mole, and more commonly between 2 and 6 Faradays per mole.

As stated heretofore, CHD can also be synthesised from mesitylene by chemical oxidation. In this process, the oxidation is effected not by the electric current but by a chemical oxidising agent, which is generally a metal salt of high valency state. The oxidation is carried out in a reaction medium having a composition falling within the general description of the anodic electrolyte solution relative to the electrochemical preparation of CHD according to the present invention. The process in which the mesitylene, completely or partly dissolved in a mixture of acetonitrile, water and sulphuric acid, is reacted with lead dioxide has proved particularly advantageous. This latter compound is used in a molar ratio varying between 0.25 and 4 with respect to the mesitylene present, whereas in the case of all the other components the indications already given regarding electrochemical oxidation are valid. The reaction temperature is between 20°C and the boiling point of the reaction mixture.

## Example 1

Oxidation of mesitylene in acetonitrile/$H_2O$/$H_2SO_4$ on a graphite anode

A cell is used provided with a cation exchange membrane, with a graphite anode, and a stainless steel cathode, each of area 100 cm$^2$. The anodic solution consists of 200 ml of $H_2O$, 5 ml of 96% $H_2SO_4$, 850 ml of acetonitrile, and 8.64 g of mesitylene. The cathodic solution consists of 5 ml of 96% $H_2SO_4$ dissolved in 1 litre of water.

Electrolysis is effected at 8°C with a current of 0.7 A for a time of 5.5 hours. On termination of electrolysis, the solution is partly neutralised to pH 4. Acetonitrile and the unreacted mesitylene (4.47 g) are distilled off.

The remaining aqueous phase is extracted with ether or another suitable solvent, which is then evaporated.

On distillation under vacuum, 2.91 g of product are obtained containing 82% by weight of CHD, and 0.87 g of high boiling products as residue. The product obtained can be purified by the normal methods (distillation or crystallisation) to obtain CHD of 99% purity.

In this electrolysis, the selectivity and current yield are 45% and 43% respectively.

## Example 2

Oxidation of mesitylene in acetonitrile/$H_2O$/phosphate-fluoroborate on a graphite anode

The apparatus described in Example 1 is used to electrolyse 17.28 g of mesitylene dissolved in 2 litres of a solution containing 600 ml of $H_2O$, 2.1 g of NaOH, 7 g of $KH_2PO_4$, 66 g of tetrabutylammonium tetrafluoroborate and a sufficient quantity of acetonitrile to make up to 2 litres.

The cathode compartment contains a $HBF_4$ solution (10 g in 2 litres of $H_2O$).

Electrolysis is effected at 18°C with a current of 1.1 A for 3.5 hours. By operating as in Example 1, 6.75 g of product are obtained with a CHD concentration of 81%, plus 2.73 g of high boiling products and 7.25 g of unreacted mesitylene. The selectivity and current yield are 43% and 50% respectively.

Example 3

Oxidation of mesitylene in acetonitrile/$H_2O$/$H_2SO_4$ on a $PbO_2$ anode

The apparatus described in Example 1, but with a different anode which in this case is $PbO_2$, is used to electrolyse 8.64 g of mesitylene dissolved in a solution containing 200 ml of $H_2O$, 850 ml of acetonitrile and 10 ml of 96% $H_2SO_4$.

The cathode compartment contains a $H_2SO_4$ solution (10 ml of 96% $H_2SO_4$ in 1 litre of $H_2O$). The electrolysis is effected at 18°C with a current of 1.6 A for 2 hours 25 minutes.

Operating as in Example 1, 3.25 g of product with a CHD concentration of 83%, 4.23 g of unreacted mesitylene and 0.53 g of high boiling products are obtained.

Selectivity 48%, current yield 49%.

Example 4

Oxidation of mesitylene in a two-phase $H_2O$/acetonitrile/$H_2SO_4$ system on a $PbO_2$ anode

The apparatus described in Example 1 is used to electrolyse 8.64 g of mesitylene dissolved in a solution containing 200 ml of $H_2O$, 850 ml of acetonitrile and 5 ml of 96% $H_2SO_4$. The cathode compartment contains a solution of 20 ml of 96% $H_2SO_4$ in 1 litre of $H_2O$.

Electrolysis is effected at 8°C with a current of 0.6 A for 6 hours 25 minutes.

Operating as in Example 1, 2.69 g of product with a CHD concentration of 81%, 4.31 g of unreacted mesitylene and 0.69 g of high boiling products are obtained.

Selectivity 40%, current yield 40%.

Example 5

Oxidation of mesitylene in a two-phase $H_2O$/acetonitrile/$H_2SO_4$ system on a $PbO_2$ anode

A cell is used provided with a cation exchange membrane, a $PbO_2$ anode and a Pb cathode, each of 800 $cm^2$.

The anodic electrolyte is prepared by mixing 3.6 l of acetonitrile, 1.2 l of $H_2O$, 0.1 l of 96% $H_2SO_4$ and 216 g of mesitylene.

The cathodic electrolyte consists of a solution of 0.1 l of 96% $H_2SO_4$ in 4 l of $H_2O$.

Electrolysis is effected at 9°C with a current of 40 A for 4 hours 49 minutes. Under these conditions the solution (which separates into two phases) is kept under strong mechanical agitation.

On termination of the reaction, the reaction mixture contains 79.19 g of CHD and 79.48 g of unreacted mesitylene, which can be separated by the methods described in Example 1.

Selectivity 46%, current yield 29%.

Example 6

Oxidation of mesitylene in a two-phase $H_2O$/acetonitrile/$H_2SO_4$ system on a graphite anode

The apparatus described in Example 5, but with different electrodes (graphite anode and stainless steel cathode), is used to electrolyse a solution consisting of 173.5 g of mesitylene, 3.2 l of acetonitrile, 1 litre of $H_2O$ and 25 ml of 96% $H_2SO_4$ at 10°C, with a current of 28 A for 5 hours 32 minutes.

The cathode compartment contains a solution of 0.1 l of 96% $H_2SO_4$ in 3.5 l of $H_2O$.

As in the preceding example, the reaction mixture is two-phase. On termination of electrolysis, it contains 82 g of CHD and 28.13 g of unreacted mesitylene, which can be separated by the methods described in Example 1.

Selectivity 45%, current yield 37%.

Example 7

Oxidation of mesitylene in acetonitrile/$H_2O$/tetrabutylammonium perchlorate/sodium bicarbonate on a graphite anode

A cell is used comprising a graphite anode and stainless steel cathode (both of area 20 $cm^2$) separated by a cation exchange membrane. A solution of 1.73 g of mesitylene, 2.3 g of tetrabutylammonium perchlorate, 2.4 g of sodium bicarbonate, 30 ml of $H_2O$ and 75 ml of acetonitrile is electrolysed at 18°C with a current of 0.49 A for 1 hour 34 minutes.

The cathode compartment contains a solution of 2.4 g of sodium bicarbonate in 100 ml of $H_2O$.

On termination of the reaction, the anodic solution contains 0.94 g of unreacted mesitylene and 0.53 g of CHD, which can be separated by the methods described in Example 1.

Selectivity 53%, current yield 49%.

Example 8

Oxidation of mesitylene in acetonitrile/$H_2O$/$HBF_4$ on a graphite anode

The apparatus described in Example 1 is used to electrolyse 8.64 g of mesitylene dissolved in a solution consisting of 800 ml of acetonitrile, 200 ml of $H_2O$ and 5 ml of a 50% $HBF_4$ solution. The cathode compartment contains 1 litre of $H_2O$ and 5 ml of a 50% $HBF_4$ solution.

Electrolysis is effected with a current of 0.58 A for 4 hours 32 minutes. On termination, the solution contains 4.47 g of unreacted mesitylene and 2.98 g of CHD, which can be separated by the methods described in Example 1.

Selectivity 56%, current yield 54%.

**Claims**

1. A process for the preparation of 2,4,6-trimethyl-4-hydroxycyclohexa-2,5-diene-1-one characterized in that mesitylene is oxidized electrolytically, the reaction being carried out within a cell equipped with a diaphragm separating the anode compartment from the cathode compartment, wherein the anolite consists of acetonitrile, water and a mineral acid or a mixture of alkali metal salts and tetraalkylammonium.

2. A process as claimed in the preceding claim, characterised in that the electrochemical oxidation reaction is effected using an anode chosen from lead, lead alloys, lead dioxide deposited on lead or its alloys, on titanium or on graphite, or graphite, carbon or materials derived from these.

3. A process as claimed in claim 2, characterised in that the anode is preferably lead dioxide or graphite.

4. A process as claimed in claim 1, characterised in that the electrochemical oxidation reaction is effected using a cathode chosen from lead, steel or alloys which are inert under the reaction conditions.

5. A process as claimed in claim 1, characterised in that the reaction is effected starting from mesitylene in a volumetric ratio with respect to the anodic electrolyte solution which varies between 1:200 and 1:1.

6. A process as claimed in the preceding claim, characterised in that said volumetric ratio varies preferably between 1:100 and 1:10.

7. A process as claimed in claims 1, 5 and 6, wherein the water is present to the extent of 1 part by volume for every 0.5—100 parts by volume of solvent.

8. A process as claimed in the preceding claim, wherein the water is preferably present to the extent of one part by volume for every 2—10 parts by volume of solvent.

9. A process as claimed in claim 1, characterised in that the reaction is effected at a current density variable between 5 mA/cm$^2$ and 150 mA/cm$^2$.

10. A process as claimed in the preceding claim, characterised in that the current density varies preferably between 7 and 100 mA/cm$^2$.

11. A process as claimed in claims 1 and 4, characterised in that the cathodic electrolyte solution comprises an aqueous or hydro-organic solution of a strong electrolyte.

12. A process as claimed in claim 1, characterised in that the reaction is effected at a temperature of between 0 and 50°C.

13. A process as claimed in the preceding claim, characterised in that the reaction is preferably effected at a temperature between 5 and 25°C.

14. A process as claimed in claim 1, characterised in that the reaction is effected with a current quantity chosen between 1 and 8 Faradays per mole.

15. A process as claimed in the preceding claim, wherein the current quantity is preferably between 2 and 6 Faradays per mole.

**Patentansprüche**

1. Verfahren zur Herstellung von 2,4,6-trimethyl-4-hydroxycyclohexa-2,5-dien-1-on, dadurch gekennzeichnet, daß Mesitylen elektrochemisch oxydiert wird, daß die Reaktion in einer Zelle, die mit einem, die Anoden- und Kathodenabteile trennenden, Diaphragma versehen ist, durchgeführt wird, und daß die anodische Elektrolytlösung Acetonitril, Wasser und eine Mineralsäure, oder ein Gemisch von Alkalisalzen und Tetraalkylammonium umfaßt.

2. Verfahren gemäß dem vorhergehenden Anspruch, dadurch gekennzeichnet, daß die elektrochemische Oxydationsreaktion unter Verwendung einer Anode, ausgewählt aus Blei, Bleilegierungen, Bleioxid, abgesetzt auf Blei, oder seinen Legierungen, auf Titan oder auf Graphit, Kohlenstoff oder von diesen stammenden Materialien, durchgeführt wird.

3. Verfahren gemäß Anspruch 2, dadurch gekennzeichnet, daß die Anode vorzugsweise Bleioxid oder Graphit ist.

4. Verfahren gemäß Anspruch 1, dadurch gekennzeichnet, daß die elektrochemische Oxydationsreaktion unter Verwendung enire Kathode, ausgewählt aus Blei, Stahl oder Legierungen, welche unter den Reaktionsbedingungen inert sind, durchgeführt wird.

5. Verfahren gemäß Anspruch 1, dadurch gekennzeichnet, daß die Reaktion, ausgehend von Mesitylen in einem volumetrischen Verhältnis in Bezug auf die anodische Elektrolytlösung, das zwischen 1:200 und 1:1 variiert, durchgeführt wird.

6. Verfahren nach den vorhergehenden Anspruch, dadurch gekennzeichnet, daß das volumetrische Verhältnis vorzugsweise zwischen 1:100 und 1:10 variiert.

7. Verfahren nach den Ansprüchen 1, 5, und 6, dadurch gekennzeichnet, daß das Wasser bis zu einem Ausmaß von einem Volumteil für jede 0,5—100 Volumteile Lösungsmittel vorhanden ist.

8. Verfahren nach dem vorhergehenden Anspruch, dadurch gekennzeichnet, daß das Wasser vorzugsweise in einem Ausmaß von einem Volumteil für jede 2—10 Volumteile Lösungsmittel vorhanden ist.

9. Verfahren gemäß Anspruch 1, dadurch gekennzeichnet, daß die Reaktion bei einer Stromdichte, die

zwischen 5 mA/$cm^2$ und 150 mA/$cm^2$ variiert, durchgeführt wird.

10. Verfahren nach dem vorhergehenden Anspruch, dadurch gekennzeichnet, daß die Stromdichte vorzugsweise zwischen 5 und 100 mA/$cm^2$ variiert.

11. Verfahren nach den Ansprüchen 1 und 4, dadurch gekennzeichnet, daß die kathodische Elektrolytlösung eine wässrige oder wässrig-organische Lösung eines starken Elektrolyts umfaßt.

12. Verfahren gemäß Anspruch 1, dadurch gekennzeichnet, daß die Reaktion bei einer Temperatur von 0°C bis 50°C durchgeführt wird.

13. Verfahren nach dem vorhergehenden Anspruch, dadurch gekennzeichnet, daß die Reaktion vorzugsweise bei einer Temperature zwischen 5°C und 25°C durchgeführt wird.

14. Verfahren gemäß Anspruch 1, dadurch gekennzeichnet, daß mit einer Strommenge, ausgewählt zwischen 1 und 8 Faraday pro Mol, durchgeführt wird.

15. Verfahren nach dem vorhergehenden Anspruch, dadurch gekennzeichnet, daß die Strommenge vorzugsweise zwischen 2 und 6 Faraday pro Mol liegt.

**Revendications**

1. Procédé de préparation de la 2,4,6-triméthyl-4-hydroxycyclohexa-2,5-diène-1-one, caractérisé en ce que l'on oxyde du mésitylène par voie électrolytique, la réaction étant effectuée dans une cellule équipée d'un diaphragme séparant le compartiment anodique du compartiment cathodique, l'anolyte étant constituée d'acétonitrile, d'eau et d'un acide minéral ou d'un mélange de sel de métaux alcalins et de tétralkyl-ammonium.

2. Procédé conforme à la revendication précédente, caractérisé en ce que la réaction d'oxydation électrochimique est effectuée à l'aide d'une anode choisie parmi le plomb, les alliages de plomb, du dioxyde de plomb déposé sur du plomb ou ses alliages, sur du titane ou sur du graphite, ou bien du graphite, du carbone ou des matériaux dérivés de ceux-ci.

3. Procédé conforme à la revendication 2, caractérisé en ce que l'anode est de préférence en dioxyde de plomb ou en graphite.

4. Procédé conforme à la revendication 1, caractérisé en ce que la réaction d'oxydation électrochimique est effectuée à l'aide d'une cathode choisie parmi du plomb, de l'acier ou des alliages qui sont inertes dans les conditions de réaction.

5. Procédé conforme à la revendication 1, caractérisé en ce que l'on effectue la réaction en partant de mésitylène en un rapport volumique, par rapport à la solution anodique d'électrolyte, qui varie entre 1:200 et 1:1.

6. Procédé conforme à la revendication précédente, caractérisé en ce que ledit rapport volumique varie de préférence entre 1:100 et 1:10.

7. Procédé conforme aux revendications 1, 5 et 6, dans lequel l'eau est présente à raison de 1 partie en volume pour 0,5 à 100 parties en volume de solvant.

8. Procédé conforme à la revendication précédente, dans lequel l'eau est de préférence présente à raison de 1 partie en volume pour 2 à 10 parties en volume de solvant.

9. Procédé conforme à la revendication 1, caractérisé en ce que la réaction est effectuée avec une densité de courant variable entre 5 mA/$cm^2$ et 150 mA/$cm^2$.

10. Procédé conforme à la revendication précédente, caractérisé en ce que la densité de courant varie de préférence entre 7 et 100 mA/$cm^2$.

11. Procédé conforme aux revendications 1 et 4, caractérisé en ce que la solution cathodique d'électrolyte comprend une solution aqueuse ou hydro-organique d'un électrolyte fort.

12. Procédé conforme à la revendication 1, caractérisé en ce que la réaction est effectuée à une température située entre 0 et 50°C.

13. Procédé conforme à la revendication précédente, caractérisé en ce que la réaction est de préférence effectuée à une température située entre 5 et 25°C.

14. Procédé conforme à la revendication 1, caractérisé en ce que la réaction est effectuée avec une quantité de courant choisie entre 1 et 8 Faradays par mole.

15. Procédé conforme à la revendication précédente, dans lequel la quantité de courant est de préférence située entre 2 et 6 Faradays par mole.